# EUROPEAN PATENT SPECIFICATION

(11) **EP 0 601 708 B1**
(45) Date of publication and mention of the grant of the patent: **22.03.2000**
(21) Application number: 93308785.0
(22) Date of filing: 03.11.1993
(51) Int. Cl.: A61M 15/00, A61M 16/20

(54) **Mask for inhalation with exhalation valve**
Atemmaske mit einem Ausatmungsventil
Masque d'inhalation avec soupape d'exhalation

(30) Priority: 09.11.1992 US 973280
(43) Date of publication of application: 15.06.1994
(73) Proprietor: CANADIAN MONAGHAN LIMITED, London, Ontario N6A 4W1 (CA)
(72) Inventor: Foley, Martin P., London, Ontario N6G 3V8 (CA); Morton, Robert, London, Ontario N6G 4C1 (CA)
(74) Representative: Carpenter, David

(56) References cited:
- US-A- 480 962
- US-A- 2 985 169
- US-A- 3 232 292
- US-A- 4 832 015
- US-A- 5 012 803
- US-A- 5 109 839
- US-A- 5 186 165

## Description

Breathing problems due to allergies, asthma, etc. are widespread. It is known that such problems can be helped with inhalation of appropriate medication, such as a beta agonist. Small cartridges containing such medication are provided. Each cartridge has a valve which when activated dispenses a predetermined quantity of medication as a spray. Such devices are known as metered dose inhalers (MDI). Such metered dose inhalers are rather inefficient in delivering the medication to the patient. It is known that provision of some sort of an inhalation chamber between the MDI and the patient's mouth materially improves delivery to the patient. One such device that has met rather considerable commercial success is disclosed and claimed in Nowacki et al U.S. Patent 4,470,412.

Further problems are encountered with delivery of antiasthmatic medication to children. With adults in otherwise reasonably good health the patient generally can be relied upon to handle the matter himself, or to communicate with a healthcare provider. However, children, particularly infants, cannot readily follow directions, and often cannot communicate with a healthcare provider. Accordingly, efforts have been made so that a healthcare provider can readily observe whether a small child or infant is properly inhaling and exhaling, and thereby taking in the necessary medication. Two inhalers for this purpose are shown in Nowacki et al U.S. Patent 4,809,692, and in Nowacki et al U.S. Patent 4,832,015. It has been found in practice that anxious mothers often produce false readings with infants and other small children, and it further has been found that producing requisite plastic moldings at a commercially acceptable cost has been difficult.

In the last two US patents noted above a small mask is attached to the exit end of the aerosolizing chamber to engage an infant's face to ensure proper movement of the vaporized or aerosolized medication from the chamber into the patient's mouth and nose. Such mask is generally made of a plastic or rubber material. In the first of these two patents a whistle is provided that operates upon inhalation or exhalation of the patient (or both) so that a sound will be produced that can be observed by a healthcare provider. However, the sound is not very loud, and is sometimes indiscernable in conditions of relatively high ambient noise level. In the second of such two patents a bubble of thinner material is formed integral with the mask, and is intended to move in and out with inhalation and exhalation. The bubble must be thin enough to flex readily, but not so thin as to tear or otherwise fracture. Molding of the mask to produce a relatively thick mask, and the extremely thin integral bubble is difficult.

It will be recognised that a person who is elderly, or who is sick, or who is in some manner incapacitated may present many of the same problems of communicating with or being observed by a healthcare provider as with infants.

According to the invention there is provided a mask for the inhalation of medication by a human being, said mask being molded of plastic material or the like having an interior and an opening in a front portion adapted to receive a hollow body having air with medication dispersed therein, a sidewall expanding outwardly from said front portion to a rear portion adapted to fit sealingly on a human face and covering the mouth and nose, and a transverse wall wherein is located a normally closed one-way valve opening on exhalation to pass exhaled air from said mask and closing in the absence of exhaled air to prevent entry of outside air.

In such a mask, the valve is closed upon rest or upon inhalation, but discernably moves to an open position upon exhalation by the patient.

In carrying out the principles of the present invention a pediatric mask is provided such as in US Patents 4,809,692 and 4,832,015 mentioned above. The preferred material for moulding such mask is silicone rubber. This material can be autoclaved for sterilization, and is well accepted by the medical profession and governmental bodies that might have to approve of the mask. The mask is translucent, and hence it is possible to see at least a limited distance therethrough. In a preferred form of the invention a valve member is also molded of silicone rubber and is assembled with the balance of the mask by means of an insert and pull operation, with no added fastener being required. In a second form of the invention the valve is molded as an integral part of the mask. Other types of observable one-way air valves are contemplated but the two herein are sufficient for illustration.

The invention will best be understood with reference to the following specification when taken in connection with the accompanying drawings wherein:
Fig. 1 is a side view of a preferred form of mask having an exhalation valve therein;
Fig. 2 is a view taken from the right side of Fig. 1, comprising an end view of the mask;
Fig. 3 is a view similar to Fig. 2 but before installation of the valve;
Fig. 4 is a sectional view of the valve and a portion of the mask on an enlarged scale as taken along the line 4-4 in Fig. 3;
Fig. 5 is an end view of the valve and adjacent portion of the mask as taken from the right end of Fig. 4 on a further enlarged scale;
Fig. 6 is a perspective view of the mask;
Fig. 7 is a side view on an enlarged scale of the closure member of the valve;
Fig. 8 is a view of the valve closure member as taken from the right side of Fig. 7;
Fig. 9 is an axial sectional view of a second embodiment of the mask;
Fig. 10 is a perspective view of the mask of Fig. 9 as taken from above and the front end:
Fig. 11 is a fragmentary right end view of the valve portion of the mask of Fig. 9 as taken substantially along the line 11-11 in Fig. 9; and
Fig. 12 is a top view of the mask of Fig. 9.

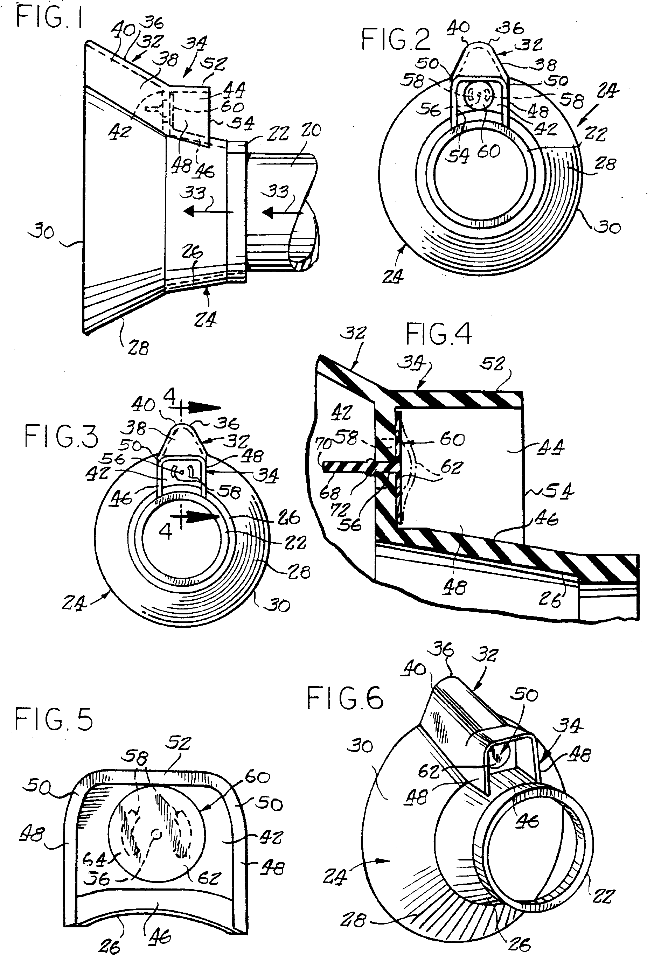

Turning now to the drawings in greater particularity, and first to Figs. 1-8, there will be seen a cylindrical aerosolization chamber 20 (Fig. 1). This chamber is only shown in part, since it may be the same as that shown in Nowacki et al 4,470,412 or in Foley et al 5,012,803, except that the exhalation ports in the aerosolization chamber are deleted. The aerosolization chamber is molded of a semi-rigid plastic, and the exit end thereof is inserted within a gripping ring 22 at the upstream end of a mask 24 molded of suitable material which is sufficiently pliable to conform to an infant's face. A preferred material is translucent silicone rubber. The mask includes a first frustoconical portion 26 of rather shallow taper integral with the ring 22, and a second or downstream portion 28 of frustoconical shape of greater taper. The outer edge 30 (the left edge in Fig. 1) is open, and air and medication flow through the chamber 20 and into the mask as indicated by the arrows 33. The inner surface of the ring 22 is cylindrical and is of small enough diameter to grip the rear end of the aerosolization chamber 20. The mask is circular in outline or cross section, with the exception of a nosepiece 32 and a valve 34, both of which are formed integral with the mask save for an operating or closure member in the valve to be noted hereinafter. The nosepiece 32 extends substantially from end to end of the rearward or portion of greater taper 28, with the frustoconical portion 28 opening radially into the nosepiece. The nosepiece has an outer edge 36 substantially parallel to the corresponding outer surface of the frustoconical portion 28 adjacent to the nosepiece. The nosepiece is substantially an inverted V having legs 38 joined together by an arcuate portion 40 which includes the outer edge 36. At the forward or upstream end the lowest portion of nosepiece 32 is terminated by a vertical wall 42. (The use of "upstream" is intended to refer relative to the air and medication movement through the aerosolization chamber 20 and into the mask 24, as indicated by the arrows 33.)

Forwardly or upstream of the wall 42 there is a boxlike housing 44 extending outwardly from the mask body and closed off therefrom by a portion of the first frustoconical portion 26 and forming a floor 46 to the valve body or housing. Walls 48 extend substantially vertically outwardly from the frustoconical portion 26, and taper inwardly at 50 to a flat roof 52. The upstream end of the valve body 44 is open at 54, and the downstream end of the housing is integrally sealed to the wall 42.

The wall 42 is provided with a small central opening 56 and with a pair of arcuate openings 58 concentric with and spaced horizontally from the smaller central opening 56.

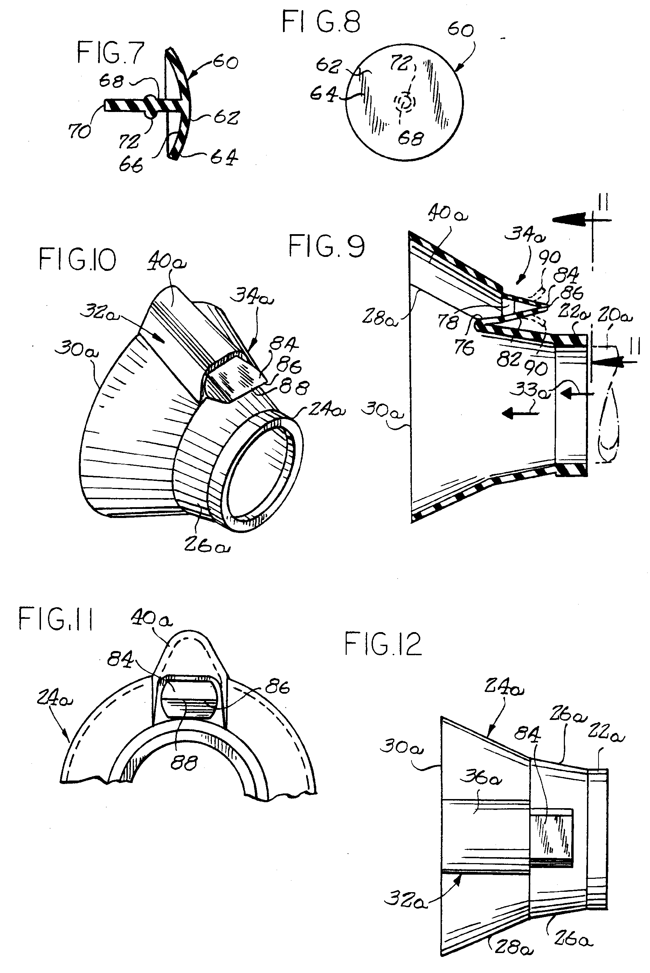

A valve closure member 60 is seen best in Figs. 7 and 8, and includes a circular head 62. The head is molded so as to be convex on the outer surface 64, and concave on the undersurface 66. Inwardly or rearwardly from the convex inner surface 66 a stem 68 extends rearwardly along the axis of the head 62. The stem somewhat closer to the undersurface 66 than to the outer end 70 of the stem is provided with an integral annular ring or enlargement 72 having a semicircular cross section.

The diameter of the stem 68 is exactly the same as the diameter of the central hole 56, and the distance from the undersurface 66 of the head 62 to the confronting edge of the enlargement 72 is the same as, or very slightly less than the thickness of the wall 42. Accordingly, to assemble the valve head 60 with the mask the end of the stem 70 is pushed through the hole 56 from the upstream surface of the wall 42. Since the enlargement 72 is spaced closer to the undersurface 66 of the valve head than it is to the outer end 70 of the stem, the outer end 70 projects through the wall 42 by the time the enlargement 72 engages the front surface of the wall. The stem adjacent the outer end 70 thus can be grasped and pulled through the hole 56. Stretching of the stem 68 somewhat reduces its diameter. The enlargement 72 is squashed inwardly by the hole 56, while the hole 56 is somewhat enlarged on a temporary basis by the enlargement 72. Thus, the enlargment 72 moves through the wall to abut the rear surface thereof as shown in Fig. 4 to hold the valve member 60 in place. The undersurface 66 of the valve head 62 is flattened against the flat front surface of the wall 42 as shown in Fig. 4. Accordingly, no air can pass through the valve structure from the upstream end (the right end in Fig. 4, for example) into the mask. However, when the infant exhales the pressure within the mask is increased. This increased pressure cannot cause backflow into the aerosolization chamber 20 due to the provision of a one-way valve near the exit end (downstream end) of the aerosolization chamber. However, the air pressure passes air through the arcuate openings 58, flexing the head 62 away from the arcuate openings 58 as shown in broken lines in Fig. 4, thereby permitting exhalation by the infant. Subsequent rest or inhalation by the infant returns the interior of the mask to ambient air pressure or below, and the valve head 62 again flattens against the wall 42.

Flexure of the valve head 62 over the openings 58 is somewhat as if there were two doors pivoted about one or more vertical axes passing through or adjacent to the smaller hole 56. Such movement readily can be seen by a healthcare provider (or the infant's mother) looking into the valve body through the open end 54, or looking through the translucent walls 48, 52 of the valve body. Thus, observation of exhalation is positively assured, and there cannot be exhalation unless there is first inhalation. Thus, it is readily ascertained that the infant is breathing and inhaling the desired medication. All of the parts are large enough to resist rupture or tearing without difficulty, and are readily molded. Installation of the valve member is quick and simple, and adds very little to the overall cost of the mask. No particular close tolerances must be held to provide an integral part of the mask that must flex as has been done in at least some of the prior art.

A modification of the invention is shown in Figs. 9-12. Many of the parts are similar to those heretofore shown and described, and are identified by the use of similar numerals with the addition of the suffix a to avoid repetition of disclosure. The mask 24a is in most respects substantially the same as that disclosed and claimed in the aforesaid U.S. Patent 4,832,015, but omits the flexible bubble previously used as an indicator of breathing by an infant.

An exhalation valve 34a is molded integrally with the remainder of the mask. It opens at its rear into the nosepiece 40a, and is of the type known as a duckbill valve. It includes a forwardly extending generally cylindrical section 76 tapering inwardly at a thinning frustoconical section 78 to a reduced diameter cylindrical section 80. The duckbill valve 34a then steps inwardly at 82 to a thinned forwardly projecting bill 84 terminating at a flat nose 86 having a slit 88 (Fig. 11) extending horizontally across it. The slit 88 is normally closed as shown by the solid line in Fig. 11. Upon inhalation pressure within the mask is reduced below ambient pressure, and the thinned side portions 84 adjacent the flat nose 86 tend to come together further to close the valve to prevent ingress of air. However, upon exhalation the slit 88 bows outwardly as shown by the broken lines 90 in Figs. 9 and 11, whereby air readily exits from the exhalation valve 34a.

Both forms of the invention as herein shown and described positively prevent ingress of air through the exhalation valve, and afford egress thereof at very little pressure above ambient pressure, less than 124 Pa (0.50 inch of water). Since the second form of the invention in Figs. 9-12 has the entire valve formed as an integral part of the mask no assembly step is required in producing the mask. However, molding is somewhat more difficult. In the first and preferred form of the mask as shown in Figs. 1-8 the assembly step is extremely simple, and does not require much additional labor. Molding is greatly simplified. As has been noted earlier the enlargement 72 on the valve stem 68 avoids the necessity of any separate fastener to hold the umbrella-like valve in installed position, yet is easily moved to installed position. The valve in the first embodiment also opens readily on exhalation with less than 124 Pa (0.50 inch of water) internal pressure above ambient, and provides positive closure against entrance of air upon inhalation. It will be appreciated that it is not desired to have air enter upon inhalation as it would dilute the medication being brought in from the aerosolization chamber 20. Exit of air through the two openings or ports 58 provides for passage of a generous amount of exhaled air.

The mask and valve are intended primarily for use with an infant or young child, and in both instances the opening of the valve is readily seen by a healthcare provider, either professional or a parent.

The mask is intended primarily for use with infants and small or young children. Approximate dimensions for the mask are just over 76.2 mm (3 inches) diameter across the rear, open end 30 and just over 50.8 mm (two inches) from this rear open end 30 to the front of the ring 22 gripping the aerosolization chamber. The inside diameter of the ring 22a is approximately 35.56 mm (1.4 inches), while the wall thickness is on the order of 2.03 mm (0.08 inch). The diameter of the valve head 62 is 11.18 mm (0.440 inch) and the thickness is 0.38 mm (0.015 inch). The length of the valve stem 68 is 7.11 mm (0.280 inch) from the underside of the head to the end 70. The radius of the valve stem is 1.02 mm (0.040 inch), except at the enlargement 72 where the outside diameter is 1.78 mm (0.070). The radius of the enlargement (in axial section of the stem) is 0.38 mm (0.015 inch).

As noted earlier there are some adult patients who must inhale medication, but are for one reason or another incapable of handling an aerosolization chamber and MDI personally. A similar mask of slightly larger size would be in order for such patients, with a healthcare provider or friend to observe the opening of the valve upon exhalation, and closure upon inhalation.

Particularly the exhalation valve is located directly adjacent the nostrils, and thereby forms a short path for exhalation from the mask.

The specific embodiments of the invention as herein shown and described are for illustrative purposes only. Various changes in structure will no doubt occur to those skilled in the art, and will be understood as forming a part of the present invention insofar as they fall within the scope of the appended claims.

## Claims

1. A mask for the inhalation of medication by a human being, said mask being molded of plastic material or the like having an interior, a central opening in a front portion (22) adapted to receive a hollow body (20) having air with medication dispersed therein, a sidewall (26, 28) expanding outwardly from said front portion (22) to a rear portion (30) adapted to fit sealingly on a human face and covering the mouth and nose, and a transverse wall (42) located adjacent said front portion (22) and extending laterally with respect to a longitudinal axis of the mask, said wall (42) being disposed in a region of the mask which is an upper region in use, and the mask including a normally closed one-way valve (34) located in said transverse wall (42), said normally closed one way valve (34) being arranged to open on exhalation to pass exhaled air from said mask and to close in the absence of exhaled air to prevent entry of outside air into the mask.

2. A mask as claimed in Claim 1, further characterised by a tunnel-like extension (32) projecting outwardly of said sidewall (26, 28) and adapted to overlie the user's nose, said extension (32) extending substantially from said rear portion (30) toward said front portion (22) and including said transverse wall (42).

3. A mask as claimed in Claim 1 or Claim 2, characterized in that said transverse wall (42) and valve (34) are positioned in their entirety short of the front portion (22) of said mask.

4. A mask as claimed in any one of the preceding Claims, characterized in that said valve (34) includes an exhalation opening (58) through said transverse wall (42) and an adjacent anchor opening (56) through said transverse wall (42), said valve (34) including a valve closure element (60) comprising a head (62) and an integral stem (68) of plastic material, said head (62) normally covering said exhalation opening (58) relatively toward the exterior of said mask and said stem (68) extending into said anchor opening (56) to anchor said head (62), said head (62) resiliently moving at least in part from said exhalation opening (58) upon exhalation.

5. A mask as claimed in Claim 4 when dependent upon Claim 2 characterized in that the transverse wall (42) is located adjacent the end of said nose receiving extension (32) relatively toward the mask open front portion (22).

6. A mask as claimed in Claim 4 or Claim 5, characterized in that said stem (68) extends entirely through said transverse wall (42) and has an enlargement (72) thereon engaging said transverse wall (42) opposite to said valve member head (62).

7. A mask as claimed in Claim 4, 5 or 6, characterized in that said head (62) is resilient and flexes away from said exhalation opening (58) upon exhalation.

8. A mask as claimed in any one of Claims 4 to 7, characterized in that there are two exhalation openings (58) through said transverse wall (42) and lying on opposite sides of said anchor (56), said head (62) having an undersurface (66) inherently concave and pulled substantially flat by said stem (68), said head (62) flexing outwardly upon exhalation.

9. A mask as claimed in Claim 2, characterized in that said exhalation valve (34) comprises a duckbill valve extending from said front end (54a) of said extension (32a) toward said front portion (22a) of said mask but terminating short of said front portion (22a).

10. A mask as claimed in any one of the preceding Claims, characterized by an inhalation valve which is normally closed but is arranged to open upon inhalation, the inhalation valve and exhalation valve (34) holding air with medication in said mask for most effective utilization of said medication.

## Patentansprüche

1. Maske für das Inhalieren von Medikamenten durch einen Menschen, wobei die Maske aus Plastikmaterial oder ähnlichem geformt wird, wobei sie folgende Komponenten hat: eine Innenfläche und eine Mittelöffnung in einem vorderen Abschnitt (22), der einen Hohlkörper (20) aufnehmen kann, in dem Luft mit einem Medikament dispensiert wird, eine Seitenwand (26, 28), die sich von dem vorderen Abschnitt (22) nach außen zu einem hinteren Abschnitt (30) erweitert, der dafür geeignet ist, abdichtend auf das Gesicht eines Menschen aufgesetzt zu werden und Mund und Nasen zu bedecken, und eine Querwand (42), die im Anschluß an den vorderen Abschnitt (22) angeordnet ist und seitlich im Verhältnis zur Längsachse der Maske verläuft, wobei die Querwand (42) in einem Bereich der Maske angeordnet ist, der bei der Benutzung ein oberer Bereich ist, und wobei die Maske ein normalerweise geschlossenes Einwegventil (34) einschließt, das sich in der Querwand (42) befindet, wobei das normalerweise geschlossene Einwegventil (34) so aufgebaut ist, daß es sich beim Ausatmen öffnet, um die ausgeatmete Luft aus der Maske herauszuführen, und sich beim Fehlen von ausgeatmeter Luft schließt, um den Eintritt von Außenluft in die Maske zu verhindern.

2. Maske nach Anspruch 1, außerdem gekennzeichnet durch eine tunnelartige Verlängerung (32), die von der Seitenwand (26, 28) nach außen vorsteht und dafür geeignet ist, über der Nase eines Benutzers zu liegen, wobei sich die Verlängerung (32) im wesentlichen von dem hinteren Abschnitt (30) zu dem vorderen Abschnitt (22) hin erstreckt und die Querwand (42) einschließt.

3. Maske nach Anspruch 1 oder Anspruch 2, dadurch gekennzeichnet, daß die Querwand (42) und das Ventil (34) in ihrer Gesamtheit kurz vor dem vorderen Abschnitt (22) der Maske angeordnet sind.

4. Maske nach einem der vorhergehenden Ansprüche, dadurch gekennzeichnet, daß das Ventil (34) eine Ausatmungsöffnung (58) durch die Querwand (42) und eine angrenzende Ankeröffnung (56) durch die Querwand (42) einschließt, wobei das Ventil (34) ein Ventilschließelement (60) einschließt, das einen Ventilteller (62) und einen integralen Ventilschaft (68) aus Plastikmaterial umfaßt, wobei der Ventilteller (62) normalerweise die Ausatmungsöffnung (58) im Verhältnis zur Außenseite der Maske bedeckt und der Ventilschaft (68) in die Ankeröffnung (56) hineinreicht, um den Ventilteller (62) zu verankern, wobei sich der Ventilteller (62) beim Ausatmen zumindest teilweise elastisch von der Ausatmungsöffnung (58) bewegt.

5. Maske nach Anspruch 4, in Abhängigkeit von Anspruch 2, dadurch gekennzeichnet, daß die Querwand (42) im Anschluß an das Ende der die Nase aufnehmenden Verlängerung (32) im Verhältnis zum offenen vorderen Abschnitt (22) der Maske hin angeordnet ist.

6. Maske nach Anspruch 4 oder Anspruch 5, dadurch gekennzeichnet, daß der Ventilschaft (68) vollständig durch die Querwand (42) verläuft und eine Erweiterung (72) aufweist, die gegenüber dem Ventilteller (62) des Ventilelements mit der Querwand (42) ineinandergreift.

7. Maske nach Anspruch 4, 5 oder 6, dadurch gekennzeichnet, daß der Ventilteller (62) elastisch ist und sich beim Ausatmen von der Ausatmungsöffnung (58) weg biegt.

8. Maske nach einem der Ansprüche 4 bis 7, dadurch gekennzeichnet, daß zwei Ausatmungsöffnungen (58) durch die Querwand (42) vorhanden sind und auf den gegenüberliegenden Seiten der Ankeröffnung (56) liegen, wobei der Ventilteller (62) eine inhärent konkave Unterfläche (66) hat und durch den Ventilschaft (68) im wesentlichen flach gezogen wird, wobei sich der Ventilteller (62) beim Ausatmen nach außen biegt.

9. Maske nach Anspruch 2, dadurch gekennzeichnet, daß das Ausatmungsventil (34) ein Entenschnabelventil umfaßt, das sich von dem vorderen Ende (54a) der Erweiterung (32a) bis hin zu dem vorderen Abschnitt (22a) der Maske erstreckt, aber kurz vor dem vorderen Abschnitt (22a) endet.

10. Maske nach einem der vorhergehenden Ansprüche, gekennzeichnet durch ein Einatmungsventil, das normalerweise geschlossen, aber so aufgebaut ist, daß es sich beim Einatmen öffnet, wobei das Einatmungsventil und das Ausatmungsventil (34) die Luft mit dem Medikament zur wirksamsten Nutzung des Medikaments in der Maske halten.

## Revendications

1. Masque pour l'inhalation d'un médicament par un être humain, ledit masque étant moulé à partir d'un matériau plastique ou similaire, comportant une partie interne, une ouverture centrale dans une partie avant (22), destinée à recevoir un corps creux (20) contenant de l'air avec un médicament qui y est dispersé, une paroi latérale (26, 28), se dilatant vers l'extérieur à partir de ladite partie avant (22) vers une partie arrière (30), destinée à être adaptée de façon étanche sur un visage humain et recouvrant la bouche et le nez, et une paroi transversale (42), agencée près de ladite partie avant (22) et s'étendant latéralement par rapport à l'axe longitudinal du masque, ladite paroi (42) étant agencée dans une région du masque constituant une région supérieure en service, le masque englobant une soupape unidirectionnelle normalement fermée (34), agencée dans ladite paroi transversale (42), ladite soupape unidirectionnelle normalement fermée (34) étant conçue de sorte à être ouverte lors de l'expiration, pour faire passer l'air expiré à partir dudit masque, et à être fermée en l'absence d'air d'expiration pour empêcher l'entrée de l'air extérieur dans le masque.

2. Masque selon la revendication 1, caractérisé en outre par une extension en forme de tunnel (32), débordant vers l'extérieur de ladite paroi latérale (26, 28) et destinée à être superposée au nez d'un utilisateur, ladite extension (32) s'étendant pratiquement de ladite partie arrière (30) vers ladite partie avant (22) et englobant ladite paroi transversale (42).

3. Masque selon les revendications 1 ou 2, caractérisé en ce que ladite paroi transversale (42) et ladite soupape (34) sont entièrement agencées peu éloignées de la partie avant (22) dudit masque.

4. Masque sel on l'une quelconque des revendications précédentes, caractérisé en ce que ladite soupape (34) englobe une ouverture d'expiration (58) traversant ladite paroi transversale (42) et une ouverture d'ancrage adjacente (56) traversant ladite paroi transversale (42), ladite soupape (34) englobant un élément de fermeture de la soupape (60) comprenant une tête (62) et une tige solidaire (68) en matériau plastique, ladite tête (62) recouvrant normalement ladite ouverture d'expiration (58) par rapport à l'extérieur dudit masque, ladite tige (68) s'étendant dans ladite ouverture d'ancrage (56) pour fixer ladite tête (62), ladite tête (62) étant déplacée par élasticité au moins en partie à partir de ladite ouverture d'expiration (58) lors de l'expiration.

5. Masque selon la revendication 4, dépendante de la revendication 2, caractérisé en ce que la paroi transversale (42) est agencée près de l'extrémité de ladite extension de réception du nez (32) par rapport à la partie avant ouverte du masque (22).

6. Masque selon les revendications 4 ou 5, caractérisé en ce que ladite tige (68) s'étend entièrement à travers ladite paroi transversale (42) et comporte un élargissement (72) s'engageant dans ladite paroi transversale (42) opposée à ladite tête de l'élément de soupape (62).

7. Masque selon les revendications 4, 5 ou 6, caractérisé en ce que ladite tête (62) est élastique et est fléchie à l'écart de ladite ouverture d'expiration (58) lors de l'expiration.

8. Masque selon l'une quelconque des revendications 4 à 7, caractérisé en ce qu'il comporte deux ouvertures d'expiration (58) traversant ladite paroi transversale (42) et agencées sur les côtés opposés de ladite ouverture d'ancrage (56) ladite tête (62) comportant une surface inférieure (66) intrinsèquement concave et étirée à un état pratiquement plat par ladite tige (68), ladite tête (62) étant fléchie vers l'extérieur lors de l'expiration.

9. Masque selon la revendication 2, caractérisé en ce que ladite soupape d'exhalation (34) comprend une soupape en bec de canard s'étendant de ladite extrémité avant (54a) de ladite extension (32a) vers ladite partie avant (22a) dudit masque, mais se terminant peu éloignée de ladite partie avant (22a).

10. Masque selon l'une quelconque des revendications précédentes, caractérisé par une soupape d'inhalation normalement fermée mais conçue de sorte à être ouverte lors de l'inhalation, la soupape d'inhalation et la soupape d'exhalation (34) retenant l'air avec le médicament dans ledit masque pour assurer vue une utilisation aussi efficace que possible dudit médicament.
